(19) Europäisches Patentamt
European Patent Office
Office européen des brevets

(11) **EP 2 668 605 B1**

(12) **EUROPEAN PATENT SPECIFICATION**

(45) Date of publication and mention
of the grant of the patent:
**06.03.2019 Bulletin 2019/10**

(51) Int Cl.:
***C12Q 1/686*** (2018.01)   ***C12Q 1/6806*** (2018.01)
***C12Q 1/6883*** (2018.01)

(21) Application number: **12738737.1**

(22) Date of filing: **20.01.2012**

(86) International application number:
**PCT/US2012/021955**

(87) International publication number:
**WO 2012/102945 (02.08.2012 Gazette 2012/31)**

(54) **RISK CALCULATION FOR EVALUATION OF FETAL ANEUPLOIDY**

RISIKOBERECHNUNG ZUR BEURTEILUNG EINER FÖTALEN ANEUPLOIDIE

CALCUL DE RISQUE POUR UNE ÉVALUATION D'ANEUPLOÏDIE FOETALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.01.2011 US 201161436135 P**
**09.12.2011 US 201113316154**
**28.12.2011 US 201113338963**

(43) Date of publication of application:
**04.12.2013 Bulletin 2013/49**

(73) Proprietor: **Ariosa Diagnostics, Inc.**
**San Jose, CA 95138 (US)**

(72) Inventors:
• **OLIPHANT, Arnold**
**San Jose, CA 95138 (US)**
• **SPARKS, Andrew**
**San Jose, CA 95138 (US)**
• **WANG, Eric**
**Milpitas, CA 95035 (US)**
• **STRUBLE, Craig**
**Glendale, WI 53217 (US)**

(74) Representative: **Mewburn Ellis LLP**
**City Tower**
**40 Basinghall Street**
**London EC2V 5DE (GB)**

(56) References cited:
US-A- 5 888 740    US-A1- 2003 054 386
US-A1- 2006 252 071    US-A1- 2007 087 345
US-A1- 2010 105 049    US-A1- 2010 120 076
US-A1- 2010 184 043    US-A1- 2011 151 442

• **FAN H C ET AL: "Noninvasive diagnosis of fetal aneuploidy by shotgun sequencing DNA from maternal blood", PROCEEDINGS OF THE NATIONAL ACADEMY OF SCIENCES OF USA, NATIONAL ACADEMY OF SCIENCE, WASHINGTON, DC; US, vol. 105, no. 42, 21 October 2008 (2008-10-21), pages 16266-16271, XP002613056, ISSN: 0027-8424, DOI: 10.1073/PNAS.0808319105 [retrieved on 2008-10-06]**
• **CHIU ROSSA W K ET AL: "Noninvasive prenatal diagnosis of fetal chromosomal aneuploidy by massively parallel genomic sequencing of DNA in maternal plasma", PROCEEDINGS OF THE NATIONAL ACADEMY OF SCIENCES OF USA, NATIONAL ACADEMY OF SCIENCE, WASHINGTON, DC; US, vol. 105, no. 51, 23 December 2008 (2008-12-23), pages 20458-20463, XP002620454, ISSN: 0027-8424, DOI: 10.1073/PNAS.0810641105 [retrieved on 2008-12-10]**
• **R. W. K. CHIU ET AL: "Supporting Information for XP02620454 (Title: Noninvasive prenatal diagnosis of fetal chromosomal aneuploidy by massively parallel genomic sequencing of DNA in maternal plasma)", PROCEEDINGS OF THE NATIONAL ACADEMY OF SCIENCES, vol. 105, no. 51, 10 December 2008 (2008-12-10), pages 1-17, XP055024153, ISSN: 0027-8424, DOI: 10.1073/pnas.0810641105**

EP 2 668 605 B1

- R. W. K. CHIU ET AL: "Non-invasive prenatal assessment of trisomy 21 by multiplexed maternal plasma DNA sequencing: large scale validity study", BMJ, vol. 342, no. jan11 1, 11 January 2011 (2011-01-11), pages 1-9, XP055024134, ISSN: 0959-8138, DOI: 10.1136/bmj.c7401
- ANDREW B. SPARKS ET AL: "Selective analysis of cell-free DNA in maternal blood for evaluation of fetal trisomy", PRENATAL DIAGNOSIS, vol. 32, no. 1, 6 January 2012 (2012-01-06), pages 3-9, XP055024132, ISSN: 0197-3851, DOI: 10.1002/pd.2922
- ANDREW B. SPARKS ET AL: "Noninvasive prenatal detection and selective analysis of cell-free DNA obtained from maternal blood: evaluation for trisomy 21 and trisomy 18", AMERICAN JOURNAL OF OBSTETRICS AND GYNECOLOGY, vol. 206, no. 4, 1 April 2012 (2012-04-01) , pages 319.E1-319.E9, XP055024123, ISSN: 0002-9378, DOI: 10.1016/j.ajog.2012.01.030
- FAN ET AL.: 'Sensitivity of Noninvasive Prenatal Detection of Fetal Aneuploidy from Maternal Plasma Using Shotgun Sequencing Is Limited Only by Counting Statistics.' PLOS ONE vol. 5, no. 5, May 2010, pages E10439-1 - E10439-7, XP055026436
- TONG ET AL.: 'Noninvasive Prenatal Detection of Fetal Trisomy 18 by Epigenetic Allelic Ratio Analysis in Maternal Plasma: Theoretical and Empirical Considerations.' CLINICAL CHEMISTRY vol. 52, no. 12, 2006, pages 2194 - 2202, XP002470084
- CHIU ET AL.: 'Noninvasive prenatal diagnosis of fetal chromosomal aneuploidy by massively parallel genomic sequencing of DNA in maternal plasma.' PNAS vol. 105, no. 51, 2008, USA, pages 20458 - 20463, XP002620454

## Description

## FIELD OF THE INVENTION

The invention provides a non-invasive method for calculating the risk of fetal genomic copy number variations such as aneuploidies using maternal samples including maternal blood, plasma and serum.

## BACKGROUND OF THE INVENTION

In the following discussion certain articles and processes will be described for background and introductory purposes. Nothing contained herein is to be construed as an "admission" of prior art. Applicant expressly reserves the right to demonstrate, where appropriate, that the articles and processes referenced herein do not constitute prior art under the applicable statutory provisions.

The American Congress of Obstetricians and Gynecologists (ACOG) recommends that pregnant women be offered non-invasive screening for fetal chromosomal abnormalities. As such existing screening methods exhibit false positive and negative rates in the range of 5% and 10% respectively, ACOG also recommends that patients categorized by screening as high risk for fetal aneuploidy be offered invasive testing such as amniocentesis or chorionic villus sampling. Although these invasive procedures are highly accurate, they are expensive and entail a risk of loss of normal fetus of approximately 0.5%. To address these limitations, non-invasive methods of fetal aneuploidy detection have been developed.

In particular, more recent attempts to identify aneuploidies have used maternal blood as a starting material. Such efforts have included the use of cell free DNA (cfDNA) to detect fetal aneuploidy in a sample from a pregnant female, including use of massively parallel shotgun sequencing (MPSS) to quantify precisely the increase in cfDNA fragments from trisomic chromosomes. The chromosomal dosage resulting from fetal aneuploidy, however, is directly related to the fraction of fetal cfDNA. Variation of fetal nucleic acid contribution between samples can thus complicate the analysis, as the level of fetal contribution to a maternal sample will vary the amounts needed to be detected for calculating the risk that a fetal chromosome is aneuploid.

For example, a cfDNA sample containing 4% DNA from a fetus with trisomy 21 should exhibit a 2% increase in the proportion of reads from chromosome 21 (chr21) as compared to a normal fetus. Distinguishing a trisomy 21 from a normal fetus with high confidence using a maternal sample with a fetal nucleic acid percentage of 4% requires a large number (>93K) of chromosome 21 observations, which is challenging and not cost-effective using non-selective techniques such as MPSS.

Thus, improved processes for the calculation of the risk of fetal genomic copy number variations, e.g.,

chromosomal dosage abnormalities such as aneuploidies, would be of great benefit in the art.

## SUMMARY OF THE INVENTION

This Summary is provided to introduce a selection of concepts in a simplified form that are further described below in the Detailed Description. This Summary is not intended to identify key or essential features of the claimed subject matter, nor is it intended to be used to limit the scope of the claimed subject matter. Other features, details, utilities, and advantages of the claimed subject matter will be apparent from the following written Detailed Description including those aspects illustrated in the accompanying drawings and defined in the appended claims.

Thus, in one embodiment the present invention provides a non-invasive method to calculate a risk of fetal aneuploidy in a maternal sample as set out in claim 1.

In some aspects of this embodiment, the maternal sample is a cell free maternal sample, and in some emdodiments, the cell free maternal sample is maternal plasma or serum. In yet other aspects, the maternal sample comprises cells.

In some aspects of this embodiment, the data on prior risk of aneuploidy comprises information related to maternal age, and in some aspects, the data on prior risk of aneuploidy comprises information related to gestational age. In yet other embodiments, the data on prior risk of aneuploidy comprises information related to both maternal age and gestational age.

In some aspects of this embodiment, the chromosome dosage of the first and second fetal chromosome is estimated by interrogating at least ten loci on each chromosome for which chromosome dosage is being estimated, and in some embodiments, the chromosome dosage of the first and second fetal chromosome is estimated by interrogating at least forty-eight loci or at least ninety-six loci on each chromosome for which chromosome dosage is being estimated.

The loci interrogated for estimation of chromosome dosage of the first and second fetal chromosome are non-polymorphic loci.

Determining the fetal nucleic acid proportion in the maternal sample is performed by interrogating one or more polymorphic loci in the maternal sample.

The risk of fetal aneuploidy is reported as an odds ratio, and in other aspects of the invention, the risk of fetal aneuploidy for the first fetal chromosome is based on a value of a likelihood of the first fetal chromosome being trisomic and a value of the likelihood of the first fetal chromosome being disomic. In other aspects, the risk of fetal aneuploidy for the first fetal chromosome is based on a value of a likelihood of the first fetal chromosome being monosomic and a value of the likelihood of the first fetal chromosome being disomic.

Numerous ways of determining the fetal nucleic acid proportion can be used, as described in more detail herein. In certain aspects, the fetal nucleic acid proportion is determined for a single fetal chromosome. In other aspects, the fetal nucleic acid proportion is determined for two or more fetal chromosomes. In yet other aspects, the fetal nucleic acid proportion reflects the total proportion of fetal nucleic acids in the maternal sample.

## DESCRIPTION OF THE FIGURES

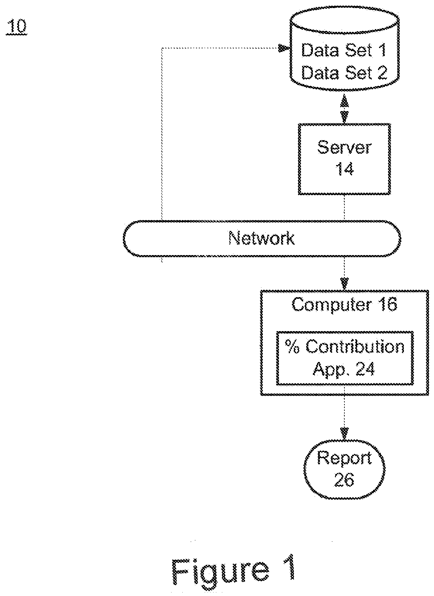

Figure 1 is a block diagram illustrating an exemplary system environment.

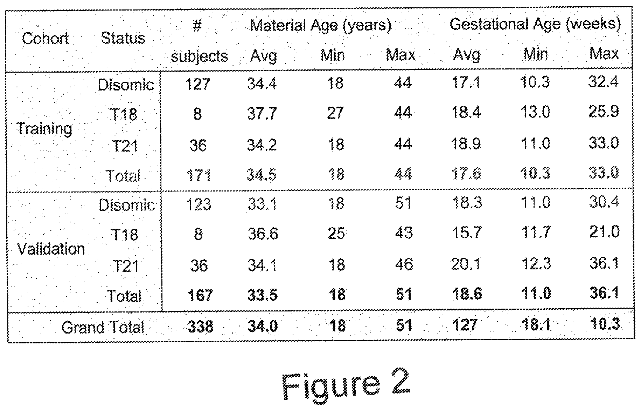

Figure 2 is a table with demographics of the subjects from which maternal samples were obtained and analyzed in the Examples.

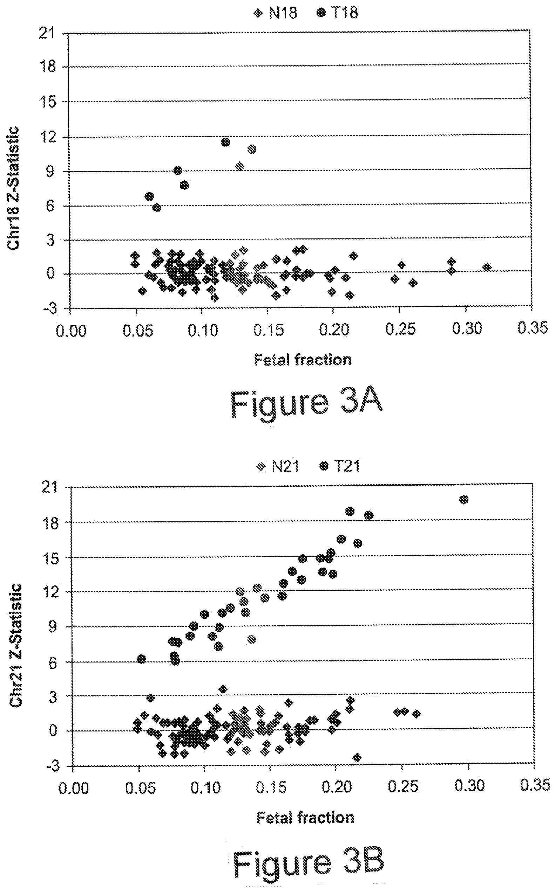

Figures 3A and 3B are graphs illustrating the cohort Z statistics versus fetal proportion. The chromosome proportion Z statistic is plotted for chromosome 18 (A) or chromosome 21 (B) versus the fraction of fetal DNA for each cohort subject. Disomic subjects are represented as black diamonds, trisomic subjects as grey diamonds.

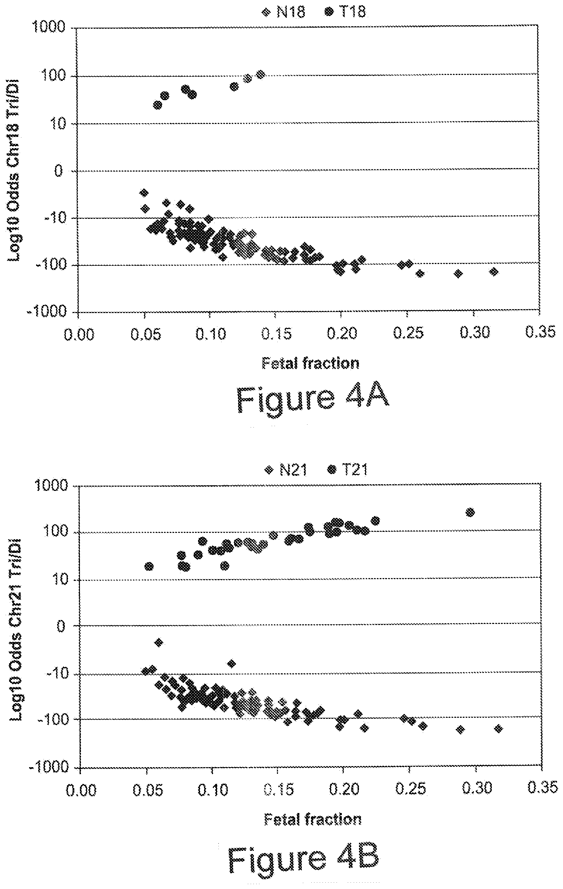

Figures 4A and 4B are graphs illustrating the cohort risk calculation odds versus fetal proportion. The risk-computed odds of trisomy versus disomy for chromosome 18 (A) or chromosome 21 (B) are plotted versus the fraction of fetal DNA for each cohort subjects. Disomic subjects are represented as black diamonds, trisomic subjects as grey diamonds.

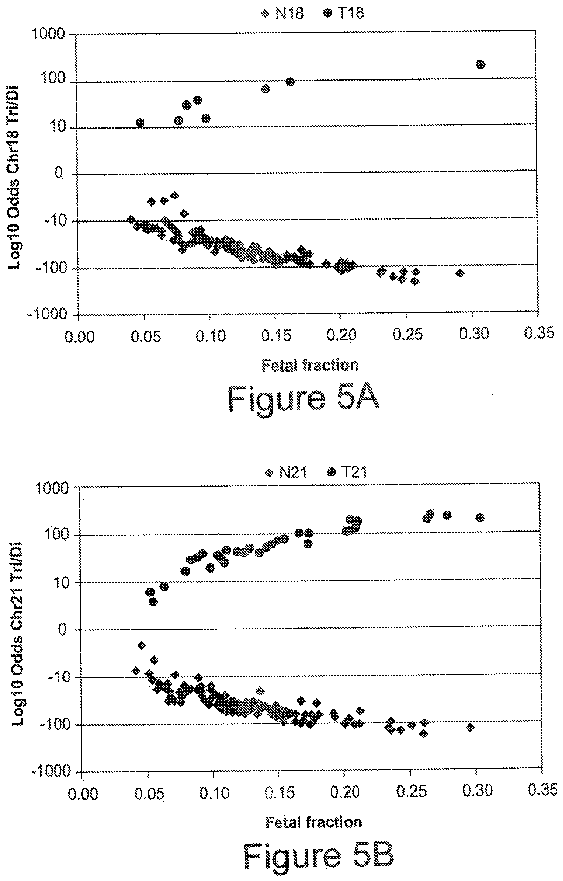

Figures 5A and 5B are graphs illustrating the blinded (second) cohort risk calculation odds of the present invention versus fetal proportion. The risk-computed odds of trisomy versus disomy for chromosome 18 (A) or chromosome 21 (B) are plotted versus the fraction of fetal DNA for each blinded (second) cohort subject. Disomic subjects are represented as black diamonds, trisomic subjects as grey diamonds.

## DETAILED DESCRIPTION OF THE INVENTION

The processes described herein may employ, unless otherwise indicated, conventional techniques and descriptions of molecular biology (including recombinant techniques), genomics, biochemistry, and sequencing technology, which are within the skill of those who practice in the art. Such conventional techniques include hybridization and ligation of oligonucleotides, next generation sequencing, and detection of hybridization using a label. Specific illustrations of suitable techniques can be had by reference to the examples herein. However, equivalent conventional procedures can, of course, also be used. Such conventional techniques and descriptions can be found in standard laboratory manuals such as Green, et al., Eds., Genome Analysis: A Laboratory Manual Series (Vols. I-IV) (1999); Weiner, et al., Eds., Genetic Variation: A Laboratory Manual (2007); Dieffenbach, Dveksler, Eds., PCR Primer: A Laboratory Manual (2003); Bowtell and Sambrook, DNA Microarrays: A Molecular Cloning Manual (2003); Mount, Bioinformatics: Sequence and Genome Analysis (2004); Sambrook and Russell, Condensed Protocols from Molecular Cloning: A Laboratory Manual (2006); and Sambrook and Russell, Molecular Cloning: A Laboratory Manual (2002) (all from Cold Spring Harbor Laboratory Press); Stryer, L., Biochemistry (4th Ed.) W.H. Freeman, New York (1995); Gait, "Oligonucleotide Synthesis: A Practical Approach" IRL Press, London (1984); Nelson and Cox, Lehninger, Principles of Biochemistry, 3rd Ed., W. H. Freeman Pub., New York (2000); and Berg et al., Biochemistry, 5th Ed., W.H. Freeman Pub., New York (2002), Before the present compositions, research tools and processes are described, it is to be understood that this invention is not limited to the specific processes, compositions, targets and uses described, as such may, of course, vary. It is also to be understood that the terminology used herein is for the purpose of describing particular aspects only and is not intended to limit the scope of the present invention, which will be limited only by appended claims.

It should be noted that as used herein and in the appended claims, the singular forms "a," "and," and "the" include plural referents unless the context clearly dictates otherwise. Thus, for example, reference to "a nucleic acid region" refers to one, more than one, or mixtures of such regions, and reference to "an assay" includes reference to equivalent steps and processes known to those skilled in the art, and so forth.

Where a range of values is provided, it is to be understood that each intervening value between the upper and lower limit of that range-and any other stated or intervening value in that stated range-is encompassed within the invention. Where the stated range includes upper and lower limits, ranges excluding either of those included limits are also included in the invention.

Unless expressly stated, the terms used herein are intended to have the plain and ordinary meaning as understood by those of ordinary skill in the art. The following definitions are intended to aid the reader in understanding the present invention, but are not intended to vary or otherwise limit the meaning of such terms unless specifically indicated.

In the following description, numerous specific details are set forth to provide a more thorough understanding of the present invention. However, it will be apparent to one of skill in the art that the present invention may be practiced without one or more of these specific details. In other instances, well-known features and procedures well known to those skilled in the art have not been described in order to avoid obscuring the invention.

## Definitions

The terms used herein are intended to have the plain and ordinary meaning as understood by those of ordinary skill in the art. The following definitions are intended to aid the reader in understanding the present invention, but are not intended to vary or otherwise limit the meaning of such terms unless specifically indicated.

The term "amplified nucleic acid" is any nucleic acid molecule whose amount has been increased at least two fold by any nucleic acid amplification or replication process performed in vitro as compared to the starting amount in a maternal sample.

The term "chromosomal dosage abnormality" refers to duplications or deletions of all (aneuploidy) or part of a chromosome.

The term "diagnostic tool" as used herein refers to any composition or assay of the invention used in combination as, for example, in a system in order to carry out a diagnostic test or assay on a patient sample.

The term "distinguishing region" refers to a region that is measurably different between loci. Such differences include, but are not limited to, single nucleotide polymorphisms (SNPs), differences in methylation status, mutations including point mutations and indels, short tandem repeats, copy number variants, and the like.

The term "hybridization" generally means the reaction by which the pairing of complementary strands of nucleic acid occurs. DNA is usually double-stranded, and when the strands are separated they will re-hybridize under the appropriate conditions. Hybrids can form between DNA-DNA, DNA-RNA or RNA-RNA. They can form between a short strand and a long strand containing a region complementary to the short one. Imperfect hybrids can also form, but the more imperfect they are, the less stable they will be (and the less likely to form).

The term "extrinsic factor" includes any information pertinent to the calculation of an odds ratio that is not empirically derived through detection of a maternal and fetal locus. Examples of such extrinsic factors include information related to maternal age, information related to gestational age, information related to previous pregnancies with an aneuploid fetus, previous serum screening results and the like. In preferred embodiments, the step of adjusting the computed odds ratio uses extrinsic factors related to both maternal age and gestational age.

The terms "locus" and "loci" as used herein refer to a nucleic acid region of known location in a genome.

The term "informative locus" as used herein refers to a locus with one or more distinguishing regions which is homozygous in one source and heterozygous in another source within a mixed sample.

The term "maternal sample" as used herein refers to any sample taken from a pregnant mammal which comprises a maternal source and a fetal source of nucleic acids (e.g., RNA or DNA).

As used herein "polymerase chain reaction" or "PCR" refers to a technique for replicating a specific piece of target DNA in vitro, even in the presence of excess nonspecific DNA. Primers are added to the target DNA, where the primers initiate the copying of the target DNA using nucleotides and, typically, Taq polymerase or the like. By cycling the temperature, the target DNA is repetitively denatured and copied. A single copy of the target DNA, even if mixed in with other, random DNA, can be amplified to obtain billions of replicates. The polymerase chain reaction can be used to detect and measure very small amounts of DNA and to create customized pieces of DNA. In some instances, linear amplification processes may be used as an alternative to PCR.

The term "polymorphism" as used herein refers to any genetic characteristic in a locus that may be indicative of that particular locus, including but not limited to single nucleotide polymorphisms (SNPs), methylation differences, short tandem repeats (STRs), and the like.

Generally, a "primer" is an oligonucleotide used to, e.g., prime DNA extension, ligation and/or synthesis, such as in the synthesis step of the polymerase chain reaction or in the primer extension techniques used in certain sequencing reactions. A primer may also be used in hybridization techniques as a means to provide complementarity of a nucleic acid region to a capture oligonucleotide for detection of a specific nucleic acid region.

The term "research tool" as used herein refers to any composition or assay of the invention used for scientific enquiry, academic or commercial in nature, including the development of pharmaceutical and/or biological therapeutics. The research tools of the invention are not intended to be therapeutic or to be subject to regulatory approval; rather, the research tools of the invention are intended to facilitate research and aid in such development activities, including any activities performed with the intention to produce information to support a regulatory submission.

The term "selected nucleic acid region" as used herein refers to a nucleic acid region corresponding to a genomic region on an individual chromosome. Such selected nucleic acid regions may be directly isolated and enriched from the sample for detection, e.g., based on hybridization and/or other sequence-based techniques, or they may be amplified using the sample as a template prior to detection of the sequence. Nucleic acids regions for use in the processing systems of the present invention may be selected on the basis of DNA level variation between individuals, based upon specificity for a particular chromosome, based on CG content and/or required amplification conditions of the selected nucleic acid regions, or other characteristics that will be apparent to one skilled in the art upon reading the present disclosure.

The terms "sequencing", "sequence determination" and the like as used herein refers generally to any and all biochemical processes that may be used to determine the order of nucleotide bases in a nucleic acid.

The term "specifically binds", "specific binding" and the like as used herein, refers to one or more molecules (e.g., a nucleic acid probe or primer, antibody, etc.) that binds to another molecule, resulting in the generation of a statistically significant positive signal under designated assay conditions. Typically the interaction will subsequently result in a detectable signal that is at least twice the standard deviation of any signal generated as a result of undesired interactions (background).

The term "value of the likelihood" refers to any value achieved by directly calculating likelihood or any value that can be correlated to or otherwise indicative of a likelihood.

The term "value of the probability" refers to any value achieved by directly calculating probability or any value that can be correlated to or otherwise indicative of a probability.

## The Invention in General

Chromosomal dosage resulting from fetal aneuploidy can be detected using nucleic acids from a maternal sample. In addition to empirical determination of the frequency of nucleic acids from a particular chromosome, the proportion of fetal nucleic acids in the maternal sample is also useful in determining the risk of fetal aneuploidy based on chromosome dosage, as it will impact the level of variation that is statistically significant in terms of the risk calculation. Utilizing such information in calculating the risk of an aneuploidy in one or more fetal chromosomes allows for a more accurate result that reflects the biological differences between samples.

## Determination of Fetal DNA Proportion in a Maternal Sample

The proportion of fetal DNA in a maternal sample is used as a part of the risk calculation of the present invention, as fetal proportion provides important information on the expected statistical presence of chromosomal dosage. Variation from the expected statistical presence may be indicative of fetal aneuploidy, an in particular a fetal trisomy or monosomy of a particular chromosome.

Any methods known in the art to estimate the percentage of fetal DNA in a maternal sample may be used, some of which are described below. Using fetal proportion as one component of the risk calculation is particularly helpful in circumstances where the level of fetal DNA in a maternal sample is low. Further, knowledge of the fetal DNA percentage may be used to determine what if any additional analyses can be performed on the sample, as it may be the case at a certain lower bound of fetal DNA percentage a system is not able to reliably perform analysis. In other aspects, determining the fetal DNA proportion in a maternal sample may additionally affect the level of certainty or power in detecting a fetal aneuploidy.

Although the following methods are described for determination of a total proportion of fetal content in a maternal sample, the proportion can also be determined on a chromosome by chromosome basis. For instance frequency information for fetal chromosome 21 can be determined as compared to fetal chromosome 18. In another example, two or more chromosomes can be used in detecting a fetal proportion, e.g., frequency of loci on chromosomes 1 and 2 can be used. In certain aspects, the chromosome used for determining fetal proportion is the chromosome interrogated for possible aneuploidy. In another aspect, the chromosome(s) used for determining fetal proportion are specifically not the chromosome interrogated for possible aneuploidy.

*Determination of Fetal DNA Content in a Maternal Sample Using Y-specific Sequences.*

In circumstances where the fetus is male, percent fetal DNA in a sample can be determined through detection of Y-specific nucleic acids and compared to maternal DNA content. For example, quantities of an amplified Y-specific nucleic acid such as a region from the sex-determining region Y gene (*SRY*), which is located on the Y chromosome and thus representative of fetal DNA in this circumstance, can be determined and compared to one or more amplified genomic regions that are present in both maternal DNA and fetal DNA (genomic regions that preferably are not from a chromosome believed to potentially be aneuploid in the fetus, e.g., an autosomal region that is not on chromosome 21, 18, or 13).

In another example, the fetal DNA concentration in a sample is calculated using methods that take into account the small percentage of background maternal DNA that may be incorrectly identified as originating from chromosome Y. Specifically, using certain bioinformatics algorithms, a small number of DNA molecules are incorrectly identified as originating from chromosome Y in pregnancies with female fetuses (see, Chiu, et al., PNAS USA, 105:20458-63 (2008)). The %chrY value in a pregnancy with a male fetus is thus a composite of the amount of chromosome Y sequences contributed by the male fetus and those sequences from the maternal background DNA that are incorrectly assigned to chromosome Y. Accordingly, in certain aspects, the fetal DNA concentration can be more correctly derived from the equation: chrY% = 0.157F + 0.007(1-F) (see, Chiu, et al., BMJ, 342:c7401 (2011)).

In a preferred aspect, amplified DNA from cell free DNA is produced by the polymerase chain reaction (PCR). Other mechanisms for amplification can be used as well as will be apparent to one skilled in the art upon reading the present disclosure, including those described in more detail herein. In particular aspects, the percentage of cell free fetal DNA in the maternal sample can be determined by PCR using serially-diluted DNA isolated from the maternal sample, which can accurately quantify the number of genomes comprising the amplified genes. For example, if a blood sample contains 100% male fetal DNA, and 1:2 serial dilutions are performed, then on average the *SRY* signal will disappear 1 dilution before an autosomal signal, since there is 1 copy of the *SRY* gene and 2 copies of an autosomal gene.

In a specific aspect, the percentage of cell free fetal DNA in maternal plasma is calculated using the following formula: percentage of cell free fetal DNA = (No.

of copies of *SRY* gene × 2 × 100)/(No. of copies of autosomal gene), where the number of copies of each gene is determined by observing the highest serial dilution in which the gene was detected. The formula contains a multiplication factor of 2, which is used to normalize for the fact that there is only 1 copy of the *SRY* gene compared to two copies of the autosomal gene in each genome, fetal or maternal.

*Determination of Fetal DNA Content in a Maternal Sample Using Autosomal Informative Loci.*

The DNA from a fetus will have approximately 50% of its loci inherited from the mother and approximately 50% its loci inherited from the father. Determining which genetic loci are contributed to the fetus from non-maternal sources (informative loci) allows the estimation of fetal DNA proportion in a maternal sample, and thus provides information used to calculate statistically significant differences in chromosomal dosages for chromosomes of interest.

In certain aspects, determination of fetal polymorphisms requires targeted SNP and/or mutation analysis to identify the presence of fetal DNA in a maternal sample. In some aspects, prior genotyping of the father and/or mother may be used. For example, the parents may have undergone genotype determination to identify disease markers, e.g., markers for disorders such as cystic fibrosis, muscular dystrophy, spinal muscular atrophy or even the status of the RhD gene. Differences in polymorphisms, copy number variants or mutations can be used to determine the percentage fetal contribution in a maternal sample.

In one preferred aspect, the percent fetal cell free DNA in a maternal sample can be quantified using multiplexed SNP detection without prior knowledge of the maternal or paternal genotype. In this aspect, two or more selected polymorphic nucleic acid regions with a known SNP in each region are used. In a preferred aspect, the selected polymorphic nucleic acid regions are located on an autosomal chromosome that is unlikely to be aneuploid, e.g., not chromosomes 21, 18, or 13. The selected polymorphic nucleic acid regions from the maternal sample (e.g., plasma) are amplified. In a preferred aspect, the amplification is universal; and in a preferred embodiment, the selected polymorphic nucleic acid regions are amplified in one reaction in one vessel. Each allele of the selected polymorphic nucleic acid regions in the maternal sample is determined and quantified. In a preferred aspect, high throughput sequencing is used for such determination and quantification.

Loci are thus identified where the maternal and fetal genotypes are different; e.g., the maternal genotype is homozygous and the fetal genotype is heterozygous. This identification of informative loci is accomplished by observing a high frequency of one allele (>80%) and a low frequency (<20% and >0.15%) of the other allele for a particular selected nucleic acid region. The use of multiple loci is particularly advantageous as it reduces the amount of variation in the measurement of the abundance of the alleles between loci. All or a subset of the loci that meet this requirement are used to determine fetal contribution through statistical analysis. In one aspect, fetal contribution is determined by summing the low frequency alleles from two or more loci together, dividing by the sum of the low and high frequency alleles and multiplying by two.

For many alleles, maternal and fetal sequences may be homozygous and identical, and as this information therefore does not distinguish between maternal and fetal DNA it is not useful in the determination of percent fetal DNA in a maternal sample. The present invention utilizes allelic information where there is a distinguishable difference between the fetal and maternal DNA (e.g., a fetal allele containing at least one allele that differs from the maternal allele) in calculations of percent fetal DNA. Data pertaining to allelic regions that are the same for maternal and fetal DNA are thus not selected for analysis, or are removed from the pertinent data prior to determination of the fetal DNA proportion so as not to mask the useful data. Additional exemplary processes for quantifying fetal DNA in maternal plasma can be found, e.g., in Chu, et al., Prenat. Diagn., 30:1226-29 (2010).

In one aspect, data from selected nucleic acid regions may be excluded if the data from the region appears to be an outlier due to experimental error or from idiopathic genetic bias within a particular sample. In another aspect, selected data from certain nucleic acid regions may undergo statistical or mathematical adjustment such as normalization, standardization, clustering, or transformation prior to summation or averaging. In another aspect, data from selected nucleic acid regions may undergo both normalization and data experimental error exclusion prior to summation or averaging.

In a preferred aspect, data from 12 or more nucleic acid regions or loci are used for the analysis. In another preferred aspect, data from 24 or more nucleic acid regions or loci are used for the analysis. In another preferred aspect, data from 48 or more loci are used for the analysis. In another aspect, one or more indices are used to identify the sample, the locus, the allele or the identification of the nucleic acid. Such indices are as is described in co-pending applications US2013040375 and US2012040859.

In one preferred aspect, the percentage fetal contribution in a maternal sample is quantified using tandem SNP detection in the maternal and fetal alleles. Techniques for identifying tandem SNPs in DNA extracted from a maternal sample are disclosed in Mitchell et al, US Pat. No. 7,799,531 and U.S. Pat App Nos. US2010184043, US2010184044, US2011117548, and US2011059451. These references describe the differentiation of fetal and maternal loci through detection of at least one tandem single nucleotide polymorphism (SNP) in a maternal sample that has a different haplotype between the fetal and maternal genome. Identification and quantification of these haplotypes can be performed directly on the maternal sample and used to determine the fetal proportion of nucleic acids in the maternal sample.

*Determination of Fetal DNA Content in a Maternal Sample Using Epigenetic Allelic Ratios.*

Certain genes have been identified as having epigenetic differences between the fetus and the mother, and such genes are candidate loci for fetal DNA markers in a maternal sample. See, e.g., Chim, et al., PNAS USA, 102:14753-58 (2005). These loci, which are unmethylated in the fetus but are methylated in maternal blood cells, can be readily detected in maternal plasma. The comparison of methylated and unmethylated amplification products from a maternal sample can be used to quantify the percent fetal DNA contribution to the maternal sample by calculating the epigenetic allelic ratio for one or more of such sequences known to be differentially-methylated in fetal DNA as compared to maternal DNA.

To determine methylation status of nucleic acids in a maternal sample, the nucleic acids of the sample are subjected to bisulfite conversion. Conventional processes for such bisulphite conversion include, but are not limited to, use of commercially available kits such as the Methylamp™ DNA Modification Kit (Epigentek, Brooklyn, NY). Allelic frequencies and ratios can be directly calculated and exported from the data to determine the percentage of fetal DNA in the maternal sample.

**Empirical Estimation of Chromosome Dosage**

The dosage of fetal chromosomes used in the odds risk calculation can be estimated using a variety of techniques. The processes for detection include polymorphic detection, such as SNP detection of specific nucleic acids, or preferably non-polymorphic detection based on fetal and maternal sequences, and preferably conserved non-polymorphic sequences between the mother and fetus. These detection methods can determine both dosage of a particular chromosome, as well as the overall proportion of fetal nucleic acids in a maternal sample relative to the maternal contribution.

In estimating chromosome dosage, such frequency measurements are preferably total frequencies of the selected nucleic acid in the sample regardless of the source, and thus it is not required that the selected nucleic acids be distinguished as being from a maternal or fetal source prior to the use in the odds risk calculation.

In some aspects, nucleic acids can be selected from a maternal sample prior to detection, i.e. selectively isolated from a maternal sample prior to detection using amplification or capture techniques such as hybridization. In another specific aspect, the nucleic acids used in estimation of chromosome dosage may be selected after detection, e.g., by filtering frequency data generated from techniques such as massively parallel shotgun sequencing of nucleic acids within the maternal sample.

In some specific aspects, estimation of chromosome dosage employs selective sequencing methods that interrogate chromosome-specific loci, enabling highly multiplexed sequencing of selected loci from specific chromosomes of interest. Chromosome-selective sequencing can be used to assay simultaneously polymorphic and non-polymorphic loci in a single reaction, enabling estimation of both chromosome dosage and fetal proportion of fetal nucleic acids in the maternal sample. Subsequently, a novel risk calculation of the invention can employed, which leverages chromosome dosage and fetal proportion estimates to compute the likelihood of fetal aneuploidy (e.g., fetal trisomy) in each subject.

In one aspect, the present invention utilizes analysis of random DNA segments, such as that described in, e.g., Quake et al., US Pat Nos. 8,008,018 and 7,888,017, and Shoemaker et al., to estimate chromosome dosage (aneuploidy). Briefly, the quantity of nucleic acids within a mixed sample such as a maternal sample can be differentially detected using selected nucleic acid sequences. The nucleic acids may be genomic DNA or RNA, and preferably are mRNA. In the case of mRNA, one may choose target sequences corresponding to genes that are highly expressed in the fetus. The nucleic acids in each sample are detected with one or more sequence-specific probes directed to at least one of two target sequences in the nucleic acids to obtain a detectible reaction product. A probe specific to an interrogated chromosome is combined with the reaction sample, along with a control probe specific to another (e.g., non-interrogated) chromosome. In most cases, the reaction products will be from maternal nucleic acids, but a small number of reaction products will be from fetal nucleic acids. In order to distinguish random variation from fetal results, a large number of reactions are run, and statistical processes are applied to the results. Labeling and detection in the present process is used to distinguish the presence or absence of a single target sequence, referred to as "digital analysis," although it may be performed with sensitive nucleic acid detection processes that distinguish between one and more than one target sequence in a discrete sample.

In another example, massively parallel sequencing of nucleic acids (e.g., DNA fragments randomly selected from the sample) is used to determine the sequence of the nucleic acids in the maternal sample to determine selected frequency of the nucleic acids within the maternal sample. For detection of a chromosome frequency abnormality (e.g., a trisomy), the sequenced nucleic acids are identified as being from a first chromosome, and the total amounts of nucleic acids from at least one first chromosome in the maternal sample are compared to total amounts of nucleic acids from at least one second chromosome in the maternal sample. The total nucleic acid amounts include the nucleic acids from both the fetus and mother in the maternal sample, and the nucleic acids from the fetus are not differentiated from the maternal in determining the frequency of the nucleic acids corresponding to the chromosome frequency. Where one first chromosome is presumed to be euploid, and the second chromosome is suspected to be aneuploid, the total numbers of nucleic acids for the first and second chromosomes are compared to determine the presence or absence of said aneuploidy.

In more specific aspects, the samples used for massively parallel sequencing of nucleic acids are enriched for polymorphic regions. Exemplary techniques for performing enrichment include those disclosed in, e.g., WO2011091063, WO2011091046 and US Pat Appln No. 20110230358. Briefly, a portion of a maternal sample comprising cell free DNA is amplified to augment the number of copies of the one or more polymorphic sequences in the sample, and the amplified portions of nucleic acids are then added back to the original sample for sequencing. Alternatively, the sample is subjected to whole genome sequencing to obtain a plurality of sequence tags, and the sequences of the tags are compared to the sequence of multiple reference polymorphisms.

In some aspects, the nucleic acids are sequenced using array-based hybridization processes, such as those described in U.S. Pat. Pub. No. 2011/0172111. In other aspects, the biomolecules are detected using nanopore technology detection, such as those described in U.S. Pat. Pub. No. 2011/0124518.

In another aspect, the nucleic acids are sequenced and compared using polymorphisms that differentiate between maternal and fetal alleles in a sample, using methods such as those described in U.S. Pat Nos. 7,727,720, 7,718,370, 7,598,060, 7,442,506, 7,332,277, 7,208, 274, and 6,977,162. Briefly, the methods utilize polymorphic detection to identify chromosomal abnormalities. Sequences are determined at alleles that are homozygous in the mother and heterozygous in the fetus, and a ratio for the heterozygous alleles is determined. The ratio for the heterozygous alleles is used to indicate the presence or absence of a chromosomal abnormality.

In yet another aspect, estimation of the risk of fetal aneuploidies utilizes identification of tandem polymorphisms, such as that described in, e.g., U.S, Pat. No. 7,799,531, and U.S. Pub. Nos. 2011/0117548, 2011/0059451, 2010/0184044, 2010/184043, and 2008/0020390. Briefly, tandem SNPs are detected and used to differentiate maternal and fetal alleles in a maternal sample to detect fetal chromosomal abnormalities through comparison of maternal DNA to fetal DNA.

In a preferred aspect, the estimation of chromosomal dosage utilizes selected amplification of representative loci. Such techniques are disclosed in, e.g., US Appln Nos. US2012034603, US2013040375, US2012040859, and US2012034685. These techniques utilize detection of genomic regions using fixed sequence oligonucleotides and joining the fixed sequence oligonucleotides via ligation and/or extension. This can be accomplished using a combination of ligation and amplification, e.g., the ligation of two or more fixed sequence oligonucleotides and optionally a bridging oligonucleotide that is complementary to a region between the fixed sequence oligonucleotides. In another example, this can be accomplished using a combination of extension, ligation and amplification.

In some aspects, chromosomal dosage estimations and variations for the normal population are determined from normal samples that have a similar proportion of fetal DNA. For example, an expected chromosomal dosage for trisomy in a DNA sample with a specific percent fetal cell free DNA can be calculated by adding the percent contribution from the aneuploid chromosome. The chromosomal dosage for the sample may then be compared to the chromosomal dosage for a normal fetus and to an expected chromosomal dosage if triploid to determine statistically, using the variation of the chromosomal dosage, if the sample is more likely normal or triploid, and the statistical probability that it is one or the other.

The nucleic acid regions selected for analysis in the maternal sample include in a single reaction both nucleic acid regions for determination of percent fetal contribution as well as nucleic acid regions corresponding to two or more chromosomes used to detect a chromosomal dosage abnormality. The use of a single reaction helps to minimize the risk of contamination or bias that may be introduced using separate reactions, which may otherwise skew results. In fact, the methods of the present invention are performed as multiplexed or even highly-multiplexed reactions, where both polymorphic and non-polymorphic loci (for determining percent fetal contribution and chromosome dosage, respectively) are interrogated in a single reaction for each sample. In preferred embodiments, the multiplexing assays described in US Appln Nos. US2012034603, US2013040375, US2012040859, and US2012034685 are used, as these assays query both polymorphic and non-polymorphic loci in a maternal sample in a single multiplexed reaction.

In other aspects, one or more selected nucleic acid regions may be interrogated both for determination of fetal nucleic acid proportion as well as detection of fetal aneuploidies. Utilizing the same regions for both fetal percent contribution and detection of fetal aneuploidies further aids in minimizing bias due to experimental error or contamination.

## Computer implementation of the Processes of the Invention

Figure 1 is a block diagram illustrating an exemplary system environment in which the processes of the present invention may be implemented for calculating the relevant values. The system 10 includes a server 14 and a computer 16. The computer 16 may be in communication with the server 14 through the same or different network.

According to the exemplary embodiment, the computer 16 executes a software component 24 that calculates fetal proportion and applies this information to the values of the dosage of genomic regions and/or chromosomes. In one embodiment, the computer 16 may comprise a personal computer, but the computer 16 may comprise any type of machine that includes at least one processor and memory.

The output of the software component 24 comprises a report 26 with a value of probability that a genomic region and/or a chromosome has a dosage abnormality. In a preferred aspect this report is an odds ratio of a value of the likelihood that a region or chromosome has two copies (e.g., is disomic) and a value of the likelihood that a region or chromosome has more copies (e.g., is trisomic) or less copies (e.g., is monosomic) copies. The report 26 may be paper that is printed out, or electronic, which may be displayed on a monitor and/or communicated electronically to users via e-mail, FTP, text messaging, posted on a server, and the like.

Although the normalization process of the invention is shown as being implemented as software 24, it can also be implemented as a combination of hardware and software. In addition, the software 24 for normalization may be implemented as multiple components operating on the same or different computers.

Both the server 14 and the computer 16 may include hardware components of typical computing devices (not shown), including a processor, input devices (e.g., keyboard, pointing device, microphone for voice commands, buttons, touchscreen, etc.), and output devices (e.g., a display device, speakers, and the like). The server 14 and computer 16 may include computer-readable media, e.g., memory and storage devices (e.g., flash memory, hard drive, optical disk drive, magnetic disk drive, and the like) containing computer instructions that implement the functionality disclosed when executed by the processor. The server 14 and the computer 16 may further include wired or wireless network communication interfaces for communication.

## EXAMPLES

The following examples are put forth so as to provide those of ordinary skill in the art with a complete disclosure and description of how to make and use the present invention, and are not intended to limit the scope of what the inventors regard as their invention, nor are they intended to represent or imply that the experiments below are all of or the only experiments performed. The present aspects are, therefore, to be considered in all respects as illustrative and not restrictive.

The efficiency and accuracy of identifying aneuploidies using the odds ratio calculation of the present invention is demonstrated in the below Examples, where in a blinded cohort of 167 pregnant women, including 36 T21 and 8 T18 pregnancies, the methods correctly discriminated all T21 and T18 cases from euploid cases.

**Example 1: Subjects**

Subjects were prospectively enrolled upon providing informed consent under protocols approved by institutional review boards. Subjects were required to be at least 18 years of age, at least 10 weeks gestational age, and to have singleton pregnancies. A subset of enrolled subjects, consisting of 250 women with disomic pregnancies, 72 women with trisomy 21 (T21) pregnancies, and 16 women with trisomy 18 (T18) pregnancies, was selected for inclusion in this study. The subjects were randomized into a first cohort consisting of 127 disomic pregnancies, 36 T21 pregnancies, and 8 T18 pregnancies, and a second cohort consisting of 123 disomic pregnancies, 36 T21 pregnancies, and 8 T18 pregnancies. The trisomy status of each pregnancy was confirmed by invasive testing (fluorescent in-situ hybridization and/or karyotype analysis). The trisomy status of the first cohort was known at the time of analysis; in the second cohort, trisomy status was blinded until after risk calculation analysis.

Figure 2 is a table profiling the demographics of the samples analyzed in this study. The mean maternal age of the disomic, T21, and T18 subjects was 34, 34, and 37 years, respectively. The mean gestational age of the disomic, T21, and T18 subjects was 17.7, 19.6, and 17.0 weeks. The mean maternal ages of the disomic, T21 and T18 subjects were not significantly different between the first versus second cohorts (all T-test p>0.05). Similarly, the mean gestational ages of the disomic, T21 and T18 subjects were not significantly different between the first versus second cohorts (all T-test p>0.05).

**Example 2: Analysis of non-polymorphic loci to estimate chromosome dosage**

To estimate fetal chromosome dosage, assays were designed against 576 non-polymorphic loci on each of chromosome 18 and 21, where each assay consisted of three locus-specific oligonucleotides: a left oligo with a 5' universal amplification tail, a 5' phosphorylated middle oligo, and a 5' phosphorylated right oligo with a 3' universal amplification tail. The selected loci were used to compute a chr21 dosage metric and a chr18 dosage metric for each sample. First cohort samples were analyzed to identify 384 of the 576 loci on chr21 and chr18 best able to discriminate T21 and T18 from normal samples. First, sequence counts were normalized by systematically removing sample and assay biases using median polish (see Tukey, Exploratory Data Analysis (Addison-Wesley, Reading MA, 1977) and Irzarry, et al., NAR, 31(4):e15 (2003)).

Next, the 384 loci on each chromosome exhibiting the greatest residual difference between normal and trisomy samples were identified using Z Statistics derived from individual loci for the test chromosome and all 576 loci for the comparison chromosome. The mean of counts from the 384 chr21 loci best able to discriminate T21 from normal were divided by the sum of the mean count for the 384 chr21 and mean count for all 576 chr18 loci. A chr18 proportion metric was calculated similarly as the sum of counts from the 384 chr18 loci best able to discriminate T18 from normal divided by the sum of the mean count from all 576 chr21 loci and the mean count for the 384 chr18 loci.

A standard Z test of proportions was used to compute Z statistics:

$$Z_j = \frac{p_j - p_0}{\sqrt{\frac{p_j(1 - p_j)}{n_j}}}$$

where $p_j$ is the observed proportion for a given chromosome of interest in a given sample $j$, $p_0$ is the expected proportion for the given test chromosome calculated as the median $p_j$, and $n_j$ is the denominator of the proportion metric. Z statistic standardization was performed using iterative censoring. At each iteration, the samples falling outside of three median absolute deviations were removed. After ten iterations, mean and standard deviation were calculated using only the uncensored samples. All samples were then standardized against this mean and standard deviation. The Kolmogorov-Smirnov test (see Conover, Practical Nonparametric Statistics, pp. 295-301 (John Wiley & Sons, New York, NY, 1971)) and Shapiro-Wilk's test (see Royston, Applied Statistics, 31:115-124 (1982)) were used to test for the normality of the normal samples' Z statistics.

**Example 3: Analysis of polymorphic loci to assess percent fetal contribution**

To assess fetal nucleic acid proportion in the maternal samples, assays were designed against a set of 192 SNP-containing loci on chromosomes 1 through 12, where two middle oligos differing by one base were used to query each SNP. SNPs were optimized for minor allele frequency in the HapMap 3 dataset. Duan, et al., Bioinformation, 3(3):139-41(2008); Epub 2008 Nov 9.

Assays were designed against 576 non-polymorphic loci on each of chr18 and chr21, where each assay consisted of three locus specific oligonucleotides: a left oligo with a 5' universal amplification tail, a 5' phosphorylated middle oligo, and a 5' phosphorylated right oligo with a 3' universal amplification tail. To assess fetal fraction, assays were designed against a set of 192 SNP-containing loci on chr1-12, where two middle oligos, differing by one base, were used to query each SNP. SNPs were optimized for minor allele frequency in the HapMap 3 dataset. Duan, et al., Bioinformation, 3(3):139-41(2008); Epub 2008 Nov 9.

Oligonucleotides were synthesized by IDT and pooled together to create a single multiplexed assay pool. PCR products were generated from each subject sample as previously described. Briefly, 8mL blood per subject was collected into a Cell-free DNA tube (Streck) and stored at room temperature for up to 3 days. Plasma was isolated from blood via double centrifugation and stored at minus 20°C for up to a year. cfDNA was isolated from plasma using Viral NA DNA purification beads (Dynal), biotinylated, immobilized on MyOne C1 streptavidin beads (Dynal), and annealed with the multiplexed oligonucleotide pool. Appropriately hybridized oligonucleotides were catenated with Taq ligase, eluted from the cfDNA, and amplified using universal PCR primers. PCR product from 96 independent samples was pooled and used as template for cluster amplification on a single lane of a TruSeq v2 SR flow slide (Illumina). The slide was processed on an Illumina HiSeq 2000 to produce a 56-base locus-specific sequence and a 7-base sample tag sequence from an average of 1.18M clusters/sample. Locus-specific reads were compared to expected locus sequences. An average of 1.15M reads (97%) had fewer than 3 mismatches with expected assay structures resulting in an average of 854 reads/locus/sample.

Informative polymorphic loci were defined as loci where fetal alleles differed from maternal alleles. Because the assay exhibits allele specificities exceeding 99%, informative loci were readily identified when the fetal allele proportion of a locus was measured to be between 1 and 20%. A maximum likelihood was estimated using a binomial distribution, such as that described in co-pending application US2013024127 to determine the most likely fetal proportion based upon measurements from several informative loci. The results correlated well (R2 > 0.99) with the weighted average approach presented by Chu and colleagues (see, Chu, et al., Prenat. Diagn., 30:1226-29 (2010)).

**Example 4: Aneuploidy detection using risk calculation**

The risk of aneuploidy was calculated using an odds ratio that compares a model assuming a disomic fetal chromosome and a model assuming a trisomic fetal chromosome. The distribution of differences in observed and reference proportions were evaluated using normal distributions with a mean of 0 and standard deviation estimated using Monte Carlo simulations that randomly draw from observed data. For the disomic model, p0 was used as the expected reference proportion in the simulations. For the trisomic model, p0 was adjusted on a per sample basis with the fetal proportion adjusted reference proportion $\hat{p}_j$, defined as

$$\hat{p}_j = \frac{(1 + 0.5f_j)p_0}{\left((1 + 0.5f_j)p_0\right) + (1 - p_0)}$$

where $f_j$ was the fetal proportion for sample j. This adjustment accounts for the expected increased representation of a test chromosome when the fetus was trisomic. In the simulations both p0 and $f_j$ were randomly chosen from normal distributions using their mean and standard error estimates to account for measurement variances. Simulations are executed 100,000 times. The risk score was defined as the mean trisomy versus disomy odds ratio obtained from the simulations, adjusted by multiplying the risk of trisomy associated with the subject's maternal and gestational age.

**Example 5: Results**

Chromosome proportion Z Statistics determination. In order to select loci to be used for aneuploidy detection, the subjects of the first cohort were evaluated since their aneuploidy status was known. Six normal, one T18, and one T21 samples (8/171, or 5%) did not meet QC criteria (low count, fetal proportion <3%, and/or evidence from SNPs of a non-singleton pregnancy) and were removed from the dataset. Chromosome proportion Z Statistics were computed in the remaining samples for chr18 and chr21 (Figures 3A and 3B). 120/121 (99.2%) disomic samples had Z Statistics <3; one disomic sample had a chr21 Z Statistic of 3.5. 35/35 (100%) T21 and 7/7 (100%) T18 samples had chromosome proportion Z Statistics >3. Thus, using Z Statistic analysis, the assay system utilized in the present invention exhibited 99.2% specificity and 100% sensitivity for T21, and 100% specificity and 100% sensitivity for T18.

In order to measure fetal proportion reliably, 192 assays targeting SNPs were incorporated into a multiplex assay pool. By measuring fetal proportion and chromosome proportion in the same reaction, estimates of fetal proportion from polymorphic assays were ensured to closely represent fetal proportion in the non-polymorphic assays used to assess chromosome proportion. Fetal proportion exhibited a strong correlation (R2 >0.90) with the chromosome proportion Z Statistic in aneuploid pregnancies (Figures 3A and 3B).

Importantly, the Z Statistic was not responsive to fetal proportion in normal pregnancies, reflecting a major limitation of the Z Statistic metric: samples with low Z Statistic values arise from both euploid samples and aneuploid samples with modest fetal proportion. It was reasoned that a metric that was responsive to fetal proportion in euploid as well as aneuploid pregnancies would be preferable. Thus a risk calculation was developed that leverages fetal proportion information to (1) define expected chromosome dosages for trisomic versus disomic test chromosomes, and (2) compute the odds that a sample belongs to one or the other group.

Analysis of cohort using the risk calculation. The risk calculation was used to compute the odds of trisomy versus disomy of chr18 and chr21 in each sample within the first cohort (Figures 4A and 4B). As expected, the risk calculation odds of the present invention demonstrated a response to fetal proportion in both trisomic and disomic samples, and the response magnitude was approximately equivalent in the two groups. The risk calculation of the present invention correctly discriminated all euploid from aneuploid samples, and the difference between the lowest aneuploid odds and the euploid odds exceeded 1012. All aneuploidy samples had odds >1010.

Second of risk calculation analysis on a blinded cohort. In order to test the performance of the assay and risk calculation of the present invention in an independent set of subjects, a blinded second cohort consisting of 123 normal, 36 T21, and 8 T18 pregnancies was assayed. All samples passed QC criteria and were assigned risk calculation odds scores for chr18 and chr21 (Figures 5A and 5B). As above, the risk calculation of the present invention correctly discriminated all trisomy from disomy subjects. The difference between the lowest aneuploid odds and the highest euploid odds was 103.9. All 36 T21 and 8 T18 samples had trisomy odds exceeding 102.67 (>99.8% risk of trisomy).

Current prenatal aneuploidy screening tests employ risk thresholds of approximately 1 in 300 (10-2.5) for referral to invasive testing. If this threshold were applied to the risk calculation odds for the blinded cohort, it would yield 99.2% specificity and 100% sensitivity for each chromosome. This compares favorably with current screening methods, which can entail a 5% false positive and 10% false negative rate. Moreover, because the minimum difference between the euploid and aneuploid subjects' risk calculation odds was almost four orders of magnitude for T21 and fourteen orders of magnitude for T18, a variety of thresholds produce perfect sensitivity and specificity.

By generating sequencing template from chromosome-specific assays and by producing high mapping rates, the chromosome-selective assay employed herein permits aneuploidy detection using ~1M raw reads per subject, enabling analysis of 96 subjects per sequencing lane. By contrast, MPSS evaluates the entire genome, and requires ~25M raw reads per subject, which limits sequencing throughput to 4-6 samples per lane. Thus, the present methods employing chromosome selective assays and simultaneous interrogation of polymorphic and non-polymorphic loci in multiplexed reactions enjoys a >20-fold advantage over MPSS in sequencing cost and throughput.

The present assay's capacity for genotyping individual polymorphic loci permits simultaneous determination of fetal proportion and chromosome proportion. Fetal proportion information was leveraged by imposing a QC requirement that each sample have at least 3% fetal DNA, thereby avoiding low confidence calls arising from low proportions of fetal DNA. In addition, the risk calculation algorithm was developed to produce a fetal proportion-dependent risk score indicating the odds of a sample being trisomic versus disomic.

The risk calculation analysis of the present invention differs from chromosome proportion Z Statistic analysis in several important respects. First, because 96 samples are processed in a single batch/lane, the risk calculation leverages the observed variances within and between samples in a lane, rather than estimating variance based upon information obtained from a previously-analyzed reference dataset. Thus, the risk calculation of the present invention is less susceptible to process drift and does not require external reference samples or normalizing adjustments based upon historical information.

Second, the risk calculation employed herein is responsive to fetal proportion in both the trisomic and disomic state, whereas Z Statistic is only responsive to fetal proportion in the trisomic state. As a consequence, the risk calculation of the present invention produces overall better separation of trisomic versus disomic samples. Moreover, because samples with low fetal proportion yield odds with lower magnitudes in both disomic and trisomic samples, the risk calculation of the present invention communicates a more accurate understanding of the confidence with which a call is being made in disomic samples as well as trisomic samples.

Third, because the risk of aneuploidy varies significantly with maternal and gestational age-and because incorporating these risks is standard practice in reporting screening results-the risk calculation of the present invention is designed to accommodate incorporation of age-related risks. Specifically, because both the risk computed from the chromosome-selective assay and age-related risk reflect a subject's odds of trisomy versus disomy, these risk components are readily combined. By contrast, the Z Statistic reflects the likelihood that a sample is disomic, and therefore is not readily combined with age related risks of trisomy versus disomy. One consequence of this deficiency is that the Z Statistic will exhibit different performance depending upon a subject's age. For example, an 18 year old subject at 12 weeks' gestation and with a Z Statistic of 3 is -38 times more likely to be a false positive than a 44 year old subject at 12 weeks' gestation and with the same score.

The chromosome-selective assays used herein enable highly-multiplexed sequencing of polymorphic and non-polymorphic loci from specific chromosomes of interest in up to 96 samples simultaneously. The risk calculation of the present invention analyzes resulting chromosome dosage and fetal proportion information to provide an individualized assessment of trisomy versus disomy risk which can be combined with other risk information. In this study, the risk calculation methods of the invention correctly discriminated all T21 and T18 cases from euploid cases in both a first cohort and a blinded second cohort.

## Example 6: Aneuploidy detection

The risk calculation algorithm used in calculation of the estimated risk of aneuploidy used an odds ratio comparing a mathematic model assuming a disomic fetal chromosome and a mathematic model assuming a trisomic fetal chromosome. When $x_j = p_j - p_0$ is used to describe the difference of the observed proportion $p_j$ for

sample $i$ and the estimated reference proportion $p_0$, the risk calculation algorithm used computed:

$$\frac{P(x_j \mid T)}{P(x_j \mid D)},$$

where $T$ was the trisomic model and $D$ was the disomic model. The disomic model $D$ was a normal distribution with mean 0 and a sample specific standard deviation estimated by Monte Carlo simulations as described below. The trisomic model $T$ was also a normal distribution with mean 0, determined by transforming $x_j$ to $\hat{x}_j = p_j - \hat{p}_j$, the difference between the observed proportion and a fetal fraction adjusted reference proportion as defined by:

$$\hat{p}_j = \frac{(1+0.5 f_j) p_0}{(1+0.5 f_j) p_o + (1 - p_0)},$$

where $f_j$ was the fetal fraction for sample j. This adjustment accounted for the expected increased representation of a trisomic fetal chromosome. Monte Carlo simulations were used to estimate sample specific standard deviations for disomic and trisomic models of proportion differences. Observed proportions for each sample were simulated by non-parametric bootstrap sampling of loci and calculating means, or parametric sampling from a normal distribution using the mean and standard error estimates for each chromosome from the observed non-polymorphic locus counts. Similarly, the reference proportion p0 and fetal fraction $f_j$ were simulated by non-parametric sampling of samples and polymorphic loci respectively, or chosen from normal distributions using their mean and standard error estimates to account for measurement variances. Parametric sampling was used in this study. Simulations were executed 100,000 times, and proportion differences were computed for each execution to construct the distributions. Based on the results of these simulations, normal distributions were found to be good models of disomy and trisomy.

The final risk calculation algorithm risk score is defined as:

$$\frac{P(x_j \mid T) P(T)}{P(x_j \mid D) P(D)}$$

where $P(T)/P(D)$ is the prior risk of trisomy vs. disomy. The data on prior risk of aneuploidy was taken from well-established tables capturing the risk of trisomy associated with the subject's maternal and gestational age (Nicolaides, Ultrasound Obstet Gynecol, 21:313-321 (2003)).

While this invention is satisfied by aspects in many different forms, as described in detail in connection with preferred aspects of the invention, it is understood that the present disclosure is to be considered as exemplary of the principles of the invention and is not intended to limit the invention to the specific aspects illustrated and described herein. The scope of the invention will be measured by the appended claims. The abstract and the title are not to be construed as limiting the scope of the present invention, as their purpose is to enable the appropriate authorities, as well as the general public, to quickly determine the general nature of the invention.

**Claims**

1. A non-invasive method for calculating the risk of fetal aneuploidy in a maternal sample,
   the method comprising a step of interrogating in a single multiplexed reaction (a) one or more non-polymorphic loci in the maternal sample on each chromosome for which chromosome dosage is to be estimated, and (b) one or more polymorphic loci in the maternal sample;
   the method further comprising a computer-implemented process to calculate the risk of fetal aneuploidy in a maternal sample as an odds ratio comprising:

   estimating the chromosome dosage for two or more fetal chromosomes in the maternal sample using the information obtained from interrogating non-polymorphic loci;
   determining a fetal nucleic acid proportion in the maternal sample using the information obtained from interrogating polymorphic loci;
   providing data on prior risk of aneuploidy for at least a first fetal chromosome based on extrinsic characteristics related to maternal age and/or gestational age;
   calculating a value of a likelihood that the first fetal chromosome is aneuploid by comparing the chromosome dosage of the first fetal chromosome to the chromosome dosage of a second fetal chromosome in view of the fetal nucleic acid proportion in the maternal sample and the prior risk of aneuploidy;
   calculating a value of a likelihood that the first fetal chromosome is disomic by comparing the chromosome dosage of the first fetal chromosome to the chromosome dosage of the second fetal chromosome in view of the fetal nucleic acid proportion in the maternal sample and the prior risk of aneuploidy;
   computing an odds ratio of the risk of fetal aneuploidy, which is an odds ratio of the value of the likelihood of the first chromosome being aneuploid, and the value of the likelihood of the first chromosome being disomic.

2. The method of claim 1 wherein the risk of aneuploidy is calculated as an odds ratio that compares a model assuming a disomic fetal chromosome and a model assuming a trisomic fetal chromosome,
   wherein the distribution of differences in observed and reference proportions is evaluated using normal distributions with a mean of 0 and a standard deviation estimated using Monte Carlo simulations that randomly draw from observed data,
   wherein for the disomic model ($D$) $p0$ is used as the expected reference proportion in the simulations,
   wherein for the trisomic model ($T$) $p0$ is adjusted on a per sample basis with the fetal proportion adjusted reference proportion $\hat{p}_j$ defined as

$$\hat{p}_j = \frac{(1 + 0.5f_j)p_0}{\left((1 + 0.5f_j)p_0\right) + (1 - p_0)}$$

   wherein $f_j$ is the fetal proportion for sample $j$
   wherein the risk calculation algorithm used in calculation of the estimated risk of aneuploidy as an odds ratio computes

$$\frac{P(x_j \mid T)P(T)}{P(x_j \mid D)P(D)}$$

   wherein $x_j = p_j - p0$ is used to describe the difference of the observed population $p_j$ for sample j and the estimated reference proportion $p0$, and wherein $P(T)/P(D)$ is the prior risk of trisomy vs. disomy.

3. The method of any one of the preceding claims, wherein the maternal sample is a cell free maternal sample.

4. The method of any one of the preceding claims, wherein the cell free maternal sample is maternal plasma or serum.

5. The method of claim 1 or claim 2, wherein the maternal sample comprises cells.

6. The method of any one of the preceding claims, wherein the data on prior risk of aneuploidy comprises information related to maternal age.

7. The method of any one of the preceding claims, wherein the data on prior risk of aneuploidy comprises information related to gestational age.

8. The method of any one of the preceding claims, wherein the data on prior risk of aneuploidy comprises information related to previous pregnancies with an aneuploid fetus.

9. The method of any one of the preceding claims, wherein the data on prior risk of aneuploidy comprises information relating to previous serum screening results.

10. The method of any one of the preceding claims, wherein the chromosome dosage of the first and second fetal chromosome is estimated using information obtained by interrogating at least ten loci on each chromosome for which chromosome dosage is being estimated.

11. The method of claim 10, wherein the chromosome dosage of the first and second fetal chromosome is estimated using information obtained by interrogating at least forty-eight loci on each chromosome for which chromosome dosage is being estimated.

12. The method of claim 11, wherein the chromosome dosage of the first and second fetal chromosome is estimated using information obtained by interrogating at least ninety-six loci on each chromosome for which chromosome dosage is being estimated.

13. The method of any one of the preceding claims, wherein the risk of fetal aneuploidy for the first fetal chromosome is based on a value of a likelihood of the chromosome being trisomic and a value of the likelihood of the chromosome being disomic.

14. The method of any one of claims 1 and 3 to 12, wherein the value of the risk of fetal aneuploidy for the first fetal chromosome is based on a value of a likelihood of the chromosome being monosomic and a value of the likelihood of the chromosome being disomic.

**Patentansprüche**

1. Nichtinvasives Verfahren zur Berechnung des Risikos für fötale Aneuploidie in einer Mutterprobe, wobei das Verfahren einen Schritt des Abfragens in einer einzelnen Multiplexreaktion (a) eines oder mehrerer nichtpolymorpher Loci in der Mutterprobe auf jedem Chromosom, für das eine Chromosomendosierung abzuschätzen ist, und (b) eines oder mehrerer polymorpher Loci in der Mutterprobe umfasst; wobei das Verfahren weiters einen computerimplementierten Prozess zum Berechnen des Risikos für fötale Aneuploidie in einer Mutterprobe als Chancenverhältnis umfasst, umfassend:

Abschätzen der Chromosomendosierung für zwei oder mehr fötale Chromosomen in der Mutterprobe unter Verwendung der Informationen, die aus dem Abfragen der nichtpolymorphen Loci erhalten wurden;

Bestimmen eines fötalen Nucleinsäureanteils in der Mutterprobe unter Verwendung der Informationen, die aus dem Abfragen polymorpher Loci erhalten wurden;
Bereitstellen von Daten zum vorangehenden Risiko für Aneuploidie für zumindest ein erstes fötales Chromosom basierend auf extrinsischen Merkmalen in Bezug auf das Mutteralter und/oder Gestationsalter;
Berechnen eines Werts der Wahrscheinlichkeit, dass das erste fötale Chromosom aneuploid ist, durch Vergleichen der Chromosomendosierung des ersten fötalen Chromosoms mit der Chromosomendosierung eines zweiten fötalen Chromosoms in Anbetracht des fötalen Nucleinsäureanteils in der Mutterprobe und des vorangehenden Risikos für Aneuploidie;
Berechnen eines Werts der Wahrscheinlichkeit, dass das erste fötale Chromosom disom ist, durch Vergleichen der Chromosomendosierung des ersten fötalen Chromosoms mit der Chromosomendosierung des zweiten fötalen Chromosoms in Anbetracht des fötalen Nucleinsäureanteils in der Mutterprobe und des vorangehenden Risikos für Aneuploidie;
Berechnen eines Chancenverhältnisses des Risikos für fötale Aneuploidie, das ein Chancenverhältnis des Werts der Wahrscheinlichkeit, dass das erste Chromosom aneuploid ist, und des Werts der Wahrscheinlichkeit, dass das erste Chromosom disom ist, ist.

2. Verfahren nach Anspruch 1, wobei das Risiko für Aneuploidie als Chancenverhältnis berechnet wird, das ein Modell, das von einem disomen fötalen Chromosom ausgeht, mit einem Modell, das von einem trisomen fötalen Chromosom ausgeht, vergleicht, wobei die Verteilung von Differenzen zwischen beobachteten und Bezugsanteilen unter Verwendung von Normalverteilungen mit einem Mittel von 0 bewertet wird und einer Standardabweichung unter Verwendung von Monte-Carlo-Simulationen abgeschätzt wird, die willkürlich auf beobachtete Daten zurückgreifen,
wobei für das disome Modell ($D$) $p0$ als erwarteter Bezugsanteil in den Simulationen verwendet wird,
wobei für das trisome Modell (7) $p0$ auf einer Basis pro Probe angepasst wird, wobei der an den fötalen Anteil angepasste Bezugsanteil $\hat{p}_j$ definiert ist als

$$\hat{p}_j = \frac{(1 + 0.5f_j)p_0}{\left((1 + 0.5f_j)p_0\right) + (1 - p_0)}$$

worin $fj$ der fötale Anteil für Probe $j$ ist;
worin der bei der Berechnung des geschätzten Risikos für Aneuploidie als Chancenverhältnis verwendete Risikoberechnungsalgorithmus Folgendes berechnet:

$$\frac{P(x_j \mid T)P(T)}{P(x_j \mid D)P(D)}$$

worin *xj = pj - p0* verwendet wird, um die Differenz der beobachteten Population pj für Probe j und des geschätzten Bezugsabschnitts p0 zu beschreiben, und worin P(T) / P(D) das vorangehende Risiko für Trisomie vs. Disomie ist.

3. Verfahren nach einem der vorangegangenen Ansprüche, wobei die Mutterprobe eine zellfreie Mutterprobe ist.

4. Verfahren nach einem der vorangegangenen Ansprüche, wobei die zellfreie Mutterprobe Mutterplasma oder -serum ist.

5. Verfahren nach Anspruch 1 oder 2, wobei die Mutterprobe Zellen umfasst.

6. Verfahren nach einem der vorangegangenen Ansprüche, wobei die Daten zum vorangehenden Risiko für Aneuploidie Informationen in Bezug auf das Mutteralter umfassen.

7. Verfahren nach einem der vorangegangenen Ansprüche, wobei die Daten zum vorangehenden Risiko für Aneuploidie Informationen in Bezug auf das Gestationsalter umfassen.

8. Verfahren nach einem der vorangegangenen Ansprüche, wobei die Daten zum vorangehenden Risiko für Aneuploidie Informationen in Bezug auf frühere Schwangerschaften mit einem aneuploiden Fötus umfassen.

9. Verfahren nach einem der vorangegangenen Ansprüche, wobei die Daten zum vorangehenden Risiko für Aneuploidie Informationen in Bezug auf frühere Serum-Screeningergebnisse umfassen.

10. Verfahren nach einem der vorangegangenen Ansprüche, wobei die Chromosomendosierung des ersten und zweiten fötalen Chromosoms unter Verwendung von Informationen abgeschätzt wird, die durch Abfragen von zumindest zehn Loci auf jedem Chromosom, für das eine Chromosomendosierung abgeschätzt wird, erhalten wird.

11. Verfahren nach Anspruch 10, wobei die Chromosomendosierung des ersten und zweiten fötalen Chromosoms unter Verwendung von Informationen abgeschätzt wird, die durch Abfragen von zumindest achtundvierzig Loci auf jedem Chromosom, für das eine Chromosomendosierung abgeschätzt wird, erhalten wird.

12. Verfahren nach Anspruch 11, wobei die Chromosomendosierung des ersten und zweiten fötalen Chromosoms unter Verwendung von Informationen abgeschätzt wird, die durch Abfragen von zumindest sechsundneunzig Loci auf jedem Chromosom, für das eine Chromosomendosierung abgeschätzt wird, erhalten wird.

13. Verfahren nach einem der vorangegangenen Ansprüche, wobei das Risiko für fötale Aneuploidie für das erste fötale Chromosom auf einem Wert der Wahrscheinlichkeit, dass das Chromosom trisom ist, und einem Wert der Wahrscheinlichkeit, dass das Chromosom disom ist, basiert.

14. Verfahren nach einem der Ansprüche 1 und 3 bis 12, wobei der Wert des Risikos für fötale Aneuploidie für das erste fötale Chromosom auf einem Wert der Wahrscheinlichkeit, dass das Chromosom monosom ist, und einem Wert der Wahrscheinlichkeit, dass das Chromosom disom ist, basiert.

## Revendications

1. Procédé non invasif pour calculer le risque d'aneuploïdie foetale dans un échantillon maternel, le procédé comprenant une étape consistant à interroger, dans une seule réaction multiplexe, (a) un ou plusieurs loci non polymorphiques dans l'échantillon maternel sur chaque chromosome pour lequel un dosage chromosomique doit être estimé, et (b) un ou plusieurs loci polymorphiques dans l'échantillon maternel ; le procédé comprenant en outre un procédé mis en oeuvre par ordinateur pour calculer le risque d'aneuploïdie foetale dans un échantillon maternel en tant que rapport des cotes, comprenant :

une estimation du dosage chromosomique pour deux chromosomes foetaux ou plus dans l'échantillon maternel en utilisant les informations obtenues à partir de l'interrogation des loci non polymorphiques ; la détermination d'une proportion d'acide nucléique foetal dans l'échantillon maternel en utilisant les informations obtenues à partir de l'interrogation des loci polymorphiques ; l'obtention de données sur les antécédents de risque d'aneuploïdie pour au moins un premier chromosome foetal en se basant sur des caractéristiques extrinsèques liées à l'âge maternel et/ou l'âge gestationnel ; le calcul d'une valeur de probabilité que le premier chromosome foetal soit aneuploïde en comparant le dosage chromosomique du premier chromosome foetal au dosage chromosomique d'un second chromosome foetal compte tenu de la proportion d'acide nucléique foetal dans l'échantillon maternel et des antécédents de risque d'aneuploïdie ;

le calcul d'une valeur de probabilité que le premier chromosome foetal soit disomique en comparant le dosage chromosomique du premier chromosome foetal au dosage chromosomique du second chromosome foetal compte tenu de la proportion d'acide nucléique foetal dans l'échantillon maternel et des antécédents de risque d'aneuploïdie ;

le calcul informatique d'un rapport des cotes du risque d'aneuploïdie foetale, qui est un rapport des cotes de la valeur de la probabilité que le premier chromosome soit aneuploïde, et la valeur de la probabilité que le premier chromosome soit disomique.

2.  Procédé selon la revendication 1, dans lequel le risque d'aneuploïdie est calculé en tant que rapport des cotes qui compare un modèle supposant un chromosome foetal disomique et un modèle supposant un chromosome foetal trisomique,

dans lequel la distribution des différences dans les proportions observées et de référence est évaluée en utilisant des distributions normales avec une moyenne de 0 et un écart-type estimé en utilisant des simulations de Monte-Carlo qui sont tirées de manière aléatoire à partir des données observées,

dans lequel, pour le modèle disomique ($D$), $p0$ est utilisée en tant que proportion de référence attendue dans les simulations,

dans lequel, pour le modèle trisomique ($T$), $p0$ est ajustée pour chaque échantillon avec la proportion de référence ajustée pour la proportion foetale $\hat{p}_j$ définie en tant que

$$\hat{p}_j = \frac{(1 + 0{,}5f_j)p_0}{\left((1 + 0{,}5f_j)p_0\right) + (1 - p_0)}$$

où $f_j$ est la proportion foetale pour l'échantillon $j$

où l'algorithme de calcul du risque utilisé dans le calcul du risque d'aneuploïdie estimé en tant que rapport des cotes calcule

$$\frac{P(x_j|T)P(T)}{P(x_j|D)P(D)}$$

où $x_j = p_j - p_0$ est utilisé pour décrire la différence de la population observée $p_j$ pour l'échantillon j et la proportion de référence estimée $p_0$, et où $P(T)/P(D)$ représente les antécédents de risque de trisomie vs. disomie.

3.  Procédé selon l'une quelconque des revendications précédentes, dans lequel l'échantillon maternel est un échantillon maternel acellulaire.

4.  Procédé selon l'une quelconque des revendications précédentes, dans lequel l'échantillon maternel acellulaire est du plasma ou du sérum maternel.

5.  Procédé selon la revendication 1 ou la revendication 2, dans lequel l'échantillon maternel comprend des cellules.

6.  Procédé selon l'une quelconque des revendications précédentes, dans lequel les données sur les antécédents de risque d'aneuploïdie comprennent des informations liées à l'âge maternel.

7.  Procédé selon l'une quelconque des revendications précédentes, dans lequel les données sur les antécédents de risque d'aneuploïdie comprennent des informations liées à l'âge gestationnel.

8.  Procédé selon l'une quelconque des revendications précédentes, dans lequel les données sur les antécédents de risque d'aneuploïdie comprennent des informations liées à des grossesses antérieures avec un foetus aneuploïde.

9.  Procédé selon l'une quelconque des revendications précédentes, dans lequel les données sur les antécédents de risque d'aneuploïdie comprennent des informations liées à des résultats antérieurs de dépistage sérique.

10. Procédé selon l'une quelconque des revendications précédentes, dans lequel le dosage chromosomique du premier et second chromosome foetal est estimé en utilisant des informations obtenues en interrogeant au moins dix loci sur chaque chromosome pour lequel un dosage chromosomique est estimé.

11. Procédé selon la revendication 10, dans lequel le dosage chromosomique du premier et second chromosome foetal est estimé en utilisant des informations obtenues en interrogeant au moins quarante-huit loci sur chaque chromosome pour lequel un dosage chromosomique est estimé.

12. Procédé selon la revendication 11, dans lequel le dosage chromosomique du premier et second chromosome foetal est estimé en utilisant des informations obtenues en interrogeant au moins quatre-vingt-seize loci sur chaque chromosome pour lequel un dosage chromosomique est estimé.

**13.** Procédé selon l'une quelconque des revendications précédentes, dans lequel le risque d'aneuploïdie foetale pour le premier chromosome foetal est basé sur une valeur de probabilité que le chromosome soit trisomique et une valeur de probabilité que le chromosome soit disomique.

**14.** Procédé selon l'une quelconque des revendications 1 et 3 à 12, dans lequel la valeur du risque d'aneuploïdie foetale pour le premier chromosome foetal est basée sur une valeur de probabilité que le chromosome soit monosomique et une valeur de probabilité que le chromosome soit disomique.

10

Data Set 1
Data Set 2

Server
14

Network

Computer 16

% Contribution
App. 24

Report
26

# Figure 1

| Cohort | Status | # subjects | Material Age (years) | | | Gestational Age (weeks) | | |
|---|---|---|---|---|---|---|---|---|
| | | | Avg | Min | Max | Avg | Min | Max |
| Training | Disomic | 127 | 34.4 | 18 | 44 | 17.1 | 10.3 | 32.4 |
| | T18 | 8 | 37.7 | 27 | 44 | 18.4 | 13.0 | 25.9 |
| | T21 | 36 | 34.2 | 18 | 44 | 18.9 | 11.0 | 33.0 |
| | Total | 171 | 34.5 | 18 | 44 | 17.6 | 10.3 | 33.0 |
| Validation | Disomic | 123 | 33.1 | 18 | 51 | 18.3 | 11.0 | 30.4 |
| | T18 | 8 | 36.6 | 25 | 43 | 15.7 | 11.7 | 21.0 |
| | T21 | 36 | 34.1 | 18 | 46 | 20.1 | 12.3 | 36.1 |
| | Total | 167 | 33.5 | 18 | 51 | 18.6 | 11.0 | 36.1 |
| Grand Total | | 338 | 34.0 | 18 | 51 | 127 | 18.1 | 10.3 |

Figure 2

Figure 3A

Figure 3B

Figure 4A

Figure 4B

Figure 5A

Figure 5B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2013040375 A
- US 2012040859 A
- US 7799531 B, Mitchell
- US 2010184043 A
- US 2010184044 A
- US 2011117548 A
- US 2011059451 A
- US 8008018 B, Quake
- US 7888017 B
- WO 2011091063 A
- WO 2011091046 A
- US 20110230358 A
- US 20110172111 A
- US 20110124518 A

- US 7727720 B
- US 7718370 B
- US 7598060 B
- US 7442506 B
- US 7332277 B
- US 7208274 B
- US 6977162 B
- US 20110117548 A
- US 20110059451 A
- US 20100184044 A
- US 20080020390 A
- US 2012034603 A
- US 2012034685 A
- US 2013024127 A

### Non-patent literature cited in the description

- Genome Analysis: A Laboratory Manual Series. Cold Spring Harbor Laboratory Press, 1999, vol. I-IV
- Genetic Variation: A Laboratory Manual. Cold Spring Harbor Laboratory Press, 2007
- PCR Primer: A Laboratory Manual. Cold Spring Harbor Laboratory Press, 2003
- **BOWTELL ; SAMBROOK.** DNA Microarrays: A Molecular Cloning Manual. Cold Spring Harbor Laboratory Press, 2003
- **MOUNT.** Bioinformatics: Sequence and Genome Analysis. Cold Spring Harbor Laboratory Press, 2004
- **SAMBROOK ; RUSSELL.** Condensed Protocols from Molecular Cloning: A Laboratory Manual. Cold Spring Harbor Laboratory Press, 2006
- **SAMBROOK ; RUSSELL.** Molecular Cloning: A Laboratory Manual. Cold Spring Harbor Laboratory Press, 2002
- **STRYER, L.** Biochemistry. W.H. Freeman, 1995
- **GAIT.** Oligonucleotide Synthesis: A Practical Approach. IRL Press, 1984

- **NELSON ; COX ; LEHNINGER.** Principles of Biochemistry. W. H. Freeman Pub, 2000
- **BERG et al.** Biochemistry. W.H. Freeman Pub, 2002
- **CHIU et al.** *PNAS USA,* 2008, vol. 105, 20458-63
- **CHIU et al.** *BMJ,* 2011, vol. 342, c7401
- **CHU et al.** *Prenat. Diagn.,* 2010, vol. 30, 1226-29
- **CHIM et al.** *PNAS USA,* 2005, vol. 102, 14753-58
- **TUKEY.** Exploratory Data Analysis. Addison-Wesley, 1977
- **IRZARRY et al.** *NAR,* 2003, vol. 31 (4), e15
- **CONOVER.** Practical Nonparametric Statistics. John Wiley & Sons, 1971, 295-301
- **ROYSTON.** *Applied Statistics,* 1982, vol. 31, 115-124
- **DUAN et al.** *Bioinformation,* 2008, vol. 3 (3), 139-41
- **NICOLAIDES.** *Ultrasound Obstet Gynecol,* 2003, vol. 21, 313-321